# EUROPEAN PATENT SPECIFICATION

(11) **EP 1 399 141 B1**
(45) Date of publication and mention of the grant of the patent: **26.09.2007**
(21) Application number: 02728055.1
(22) Date of filing: 24.05.2002
(51) Int. Cl.: A61K 31/221, A61P 25/02, A61P 25/04

(54) **USE OF ACETYL L-CARNITINE FOR THE PREPARATION OF A MEDICATION FOR THE PREVENTIVE THERAPY OF POST-SURGICAL PAIN**
VERWENDUNG VON L-AZETYLCARNITIN ZUR HERSTELLUNG EINES MEDIKAMENTS ZUR PRÄVENTIVEN POST-CHIRURGISCHEN SCHMERZBEHANDLUNG
UTILISATION D'ACETYL L-CARNITINE POUR LA PREPARATION D'UNE MEDICATION POUR LE TRAITEMENT PREVENTIF DE LA DOULEUR POST-OPERATOIRE

(30) Priority: 29.05.2001 IT RM20010293
(43) Date of publication of application: 24.03.2004
(73) Proprietor: SIGMA-TAU Industrie Farmaceutiche Riunite S.p.A., 00144 Roma (IT)
(72) Inventor: CALVANI, Menotti, c/o Sigma-Tau Industrie, I-00040 Pomezia (IT); MOSCONI, Luigi, c/o Sigma-Tau Industrie Farmac., I-00040 Pomezia (IT)
(74) Representative: Spadaro, Marco
(86) International application number: PCT/IT2002/000336
(87) International publication number: WO 2002/096409

(56) References cited:
- ONOFRJ M ET AL: "L-acetylcarnitine as a new therapeutic approach for peripheral neuropathies with pain." INTERNATIONAL JOURNAL OF CLINICAL PHARMACOLOGY RESEARCH, vol. 15, no. 1, 1995, pages 9-15, XP001105959 ISSN: 0251-1649
- DI GIULIO A M ET AL: "Acetyl-L-carnitine prevents substance P loss in the sciatic nerve and lumbar spinal cord of diabetic animals." INTERNATIONAL JOURNAL OF CLINICAL PHARMACOLOGY RESEARCH, vol. 12, no. 5-6, 1992, pages 243-246, XP001106182 ISSN: 0251-1649
- SCARPINI E ET AL: "Effect of acetyl-L-carnitine in the treatment of painful peripheral neuropathies in HIV+ patients." JOURNAL OF THE PERIPHERAL NERVOUS SYSTEM: JPNS. UNITED STATES 1997, vol. 2, no. 3, 1997, pages 250-252, XP001106358 ISSN: 1085-9489
- "The Merck Manual of Diagnosis and Therapy" 1999 , MERCK RESEARCH LABORATORIES XP002215512 17 page 1363 -page 1374

## Description

The present invention relates to the use of acetyl L-carnitine for the preparation of a medicament having analgesic activity for the preventive therapy of post-surgical pain.

By analgesia it is meant a condition where the stimulus of pain present in the body is not perceived as pain.

An analgesic is a medicament capable of producing analgesia, i.e. disappearance of pain, by interfering with the perception of the nociceptive stimulus without inducing anesthesia or loss of conscience.

The administration of a medicament before the pain starts is called preventive therapy for pain or pre-emptive analgesia.

By pre-emptive analgesia it is meant a therapeutical strategy involving the precocious administration of a substance before the painful event, capable of blocking the stimulus of pain before it reaches the central nervous system, thus preventing the facilitatory response raised by the nociceptive input to the spinal cord.

It is worth to mention that the efficacy of a pre-emptive analgesic medicament not only depends on the time when the administration step is carried out prior to the painful event, but also on the real capacity of the medicament to prevent alterations of the mechanisms of central sensitization related to pain.

Therefore, this concept clarifies that the efficacy of a substance depends on its being administered before the onset of the nociceptive stimulus.

The pre-emptive analgesic property of the acetyl L-carnitine is not known, while its antalgic activity in peripheral neuropathy of different aetiology has been studied. This supposed antalgic activity was attributed to the neurotrophic action of the substance, i.e. its capacity to protect the peripheral nerve and/or stimulate its regeneration.

Such use of acetyl L-carnitine is described in Clin. Drug Invest. 10(6): 317-322 of 1995. In the same study, the antalgic effect of the substance is also mentioned, but the results are not significant from a statistical point of view. Therefore, such use of acetyl L-carnitine differs from the use proposed in the present invention, since the former does not identify any analgesic effect of the said substance.

The activity on pain of acetyl L-carnitine in patients with peripheral neuropathies is described in Int J. Clin. Pharm. Res. XV(1):9-15 of 1995 with statistically significant results.

This study also differs from the present invention, since it concerns the neuropathic pain and the interventionist therapeutic approach, rather than pre-emptive (block of painful input) or preventive (before pain onset) as described in the present invention.

In Exp. Gerontol. 29(5):569-574; 1994 the use of the acetyl L-carnitine in rats subjected to cold water stress to study the mechanisms mediated by the hypothalamus-pituitary-adrenal axis in the analgesia induced by such stress.

By comparing young and old rats, this work discloses the capacity of the acetyl L-carnitine to maintain an efficient stress response in the old rats through a mechanism linked to the slowing down of the age-dependent loss of the glucocorticoid receptors located in the hippocampus, thus favoring the positive ageing of the cellular structures in terms of maintenance and function.

The above-mentioned works describe the antalgic activity of acetyl L-carnitine and its capacity of slowing down the aging process, but they neither describe nor suggest the use of the acetyl L-carnitine as an analgesic compound, particularly as pre-emptive analgesic.

Drugs for the preventive treatment of pain are already known.

The indomethacin is active both in the prevention or reduction of post-surgical pain in patients undergoing surgical interventions. The side effects of this compound are: single or multiple ulcerations of the upper gastro-intestinal tract and frontal headache (Goodman and Gilman's: The pharmacological basis of therapeutics. Hardman JG, Limbird LE; Molinoff PB, Ruddon RW, editors: McGraw-Hill, 1997).

Clonidine and ketamine, which are compounds used as pre-emptive analgesics, give rise to undesirable effects, such as sedation, anesthesia and immobility (Goodman and Gilman's: The pharmacological basis of therapeutics. Hardman JG, Limbird LE; Molinoff PB, Ruddon RW, editors: McGraw-Hill, 1997).

In the medical field, there is a great need for medicaments endowed with analgesic activity, which can be useful for the preventive treatment of pain and do not present the disadvantages of the above-mentioned medications.

It has now been found that the acetyl L-carnitine possesses analgesic activity and can be used as an agent useful for the preventive therapy of post-surgical pain.

In particular, the compound according to the present invention is used as a pre-emptive analgesic medication for the preventive therapy of post-surgical pain.

Another object of the present invention is the use of acetyl L-carnitine, or a pharmaceutically acceptable salt thereof, for the preparation of medicament for the preventive therapy of post-surgical pain.

By pharmaceutically acceptable salt of the acetyl L-carnitine it is meant any salt thereof with an acid that does not give rise to undesirable toxic effects. These acids are well known to pharmacologists and experts in pharmacy.

Non-limiting examples of these salts are: chloride, bromide, orotate, acid aspartate, acid citrate, citrate magnesium, acid phosphate, fumarate and acid fumarate, fumarate magnesium, lactate, maleate and acid maleate, mucate, acid oxalate, pamoate, acid pamoate, acid sulphate, phosphate glucose, tartrate, acid tartrate, tartrate magnesium, 2-amine ethanesulphonate, magnesium 2-amine ethanesulphonate, tartrate coline and trichloroacetate.

The following examples illustrate the invention.

### EXAMPLE 1

The anti-nociceptive profile of acetyl L-carnitine was assessed in mice by carrying out both traditional tests on analgesia, such as hot-plate test (thermal stimulus) and abdominal constriction test (chemical stimulus), and some experimental models of hyperalgesia induced by:
- intraperitoneal administration (IP) of 0.3% and 0.6% acetic acid solution;
- morphine withdrawal syndrome;
- administration of kainic acid (20 mg/kg, IP);
- administration of N-methyl-D-aspartate (1-64 µg/mouse intrathecal)

The first experiment was the hot-plate test.

Male Swiss Webster mice weighing between 22 and 30 g were used.

The test was carried out according to O'Callaghan and Holtzman in J. Pharmacol. Exp. Ther. 197: 533-537, 1976.

According to this test, a stainless steel container is to be placed in a bain-marie at a temperature of 52.5°C.

The mouse was removed from the hot plate immediately upon the first sign of painful stimulus. The maximum latency time registered on the hot plate was 45 seconds.

The values of analgesia were determined at 15 minute-intervals.

The data of Table 1(A) show that the acute subcutaneous (SC) administration of acetyl L-carnitine at a dose of 100 mg/kg did not exert any protective effect.

**TABLE 1 (A)**

| Acute SC treatment | | | | |
|---|---|---|---|---|
| Hot plate | | | | |
| Licking latency (sec) | | | | |
| | Before treatment | After treatment | | |
| | | 15 min | 30 min | 45 min |
| Physiological | 13.6±1.2 | 14.7±1.5 | 13.1±1.8 | 14.5±1.6 |
| Solution (PS) | (20) | (20) | (20) | (20) |
| Acetyl L-carnitine | 13.9±0.7 | 17.3±1.7 | 16.8±1.2 | 15.2±1.7 |
| 100 mg/kg | (30) | (30) | (30) | (30) |

| | | | | |
|---|---|---|---|---|
| Values are mean ± standard error. In brackets is reported the number of animals. | | | | |

The data of Table 1(B) referring to the animals treated with acetyl L-carnitine at a dose of 100 mg/kg, twice a day (BID), SC for 7 days show a statistically significant antinociceptive effect.

**TABLE 1 (B)**

| 7-day sub-chronic treatment (SC) | | | | |
|---|---|---|---|---|
| Hot plate | | | | |
| Licking latency (sec) | | | | |
| | Before treatment | After treatment | | |
| | | 15 min | 30 min | 45 min |
| Physiological | 16.4±1.6 | 17.5±1.5 | 15.3±1.9 | 14.9±2.1 |
| solution | (20) | (20) | (20) | (20) |
| Acetyl L- | 21.8±1.1^ | 22.1±1.8^ | 22.5±1.9^ | 22.2±1.7^ |
| carnitine 100 mg/kg BID | (41) | (41) | (41) | (41) |

| | | | | |
|---|---|---|---|---|
| Values are mean ± standard error. In brackets is reported the number of animals; ^p<0.05 compared with the group treated with PS. | | | | |

When the acetyl L-carnitine was administered for 14 days, it did not affect the intensity of the antinociceptive effect, which was the same as after 7 days, but the test failed to be statistically significant, because the stress caused by the repeated administrations (two injections/die for 14 days) made the tolerance threshold of the control rats increase by some seconds, Table 1 (C).

**TABLE 1 (C)**

| 14-day sub-chronic treatment (SC) | | | | |
|---|---|---|---|---|
| Hot plate | | | | |
| Licking latency (sec) | | | | |
| | Before treatment | After treatment | | |
| | | 15 min | 30 min | 45 min |
| Physiological | 18.7±1.7 | 19.1±2.2 | 17.9±2.4 | 18.0±2.0 |
| solution | (7) | (7) | (7) | (7) |
| Acetyl L-carnitine | 22.4±1.4 | 22.1±2.7 | 22.5±2.1 | 23.4±2.9 |
| 100 mg/kg BID | (27) | (27) | (27) | (27) |

| | | | | |
|---|---|---|---|---|
| Values are mean ± standard error. In brackets is reported the number of animals. | | | | |

The antinociceptive action of acetyl L-carnitine persisted even 7 days after the last 14 day-series of injections at a dose of 100 mg/kg BID, SC, Table 1 (D).

**TABLE 1 (D)**

| 7 days after the end of the 14-day-treatment | | | | |
|---|---|---|---|---|
| Hot plate | | | | |
| Licking latency (sec) | | | | |
| | Before treatment | After treatment | | |
| | | 15 min | 30 min | 45 min |
| Physiological | 14.0±1.2 | 15.1±2.0 | 13.8±2.5 | 13.0±2.1 |
| solution | (7) | (7) | (7) | (7) |
| Acetyl L-carnitine | 19.2±1.6^ | 21.1±1.9^ | 18.5±1.8 | 19.0±2.3 |
| 100 mg/kg BID | (12) | (12) | (12) | (12) |

| | | | | |
|---|---|---|---|---|
| Values are mean ± standard error. In brackets is reported the number of animals; ^p<0.05 compared with the group treated with PS. | | | | |

In this case, the pain threshold of the control rats returned to the initial values and, therefore the increase of the licking latency was found to be again statistically significant.

In the same test, the acetyl L-carnitine did not affect the pain threshold after 1-8 hours from one single administration of 100 mg/kg SC, Table 2.

**TABLE 2**

| SC treatment | | | | | | | |
|---|---|---|---|---|---|---|---|
| Hot plate | | | | | | | |
| Licking latency (sec) | | | | | | | |
| | Before treatment | After treatment | | | | | |
| | | 1h | 2h | 3h | 4h | 6h | 8h |
| Physiological | 14.1 | 14.9 | 13.7 | 13.8 | 14.0 | 13.3 | 14.6 |
| solution | ±1.0 | ±1.7 | ±1.9 | ±1.4 | ± 1.6 | ±1.5 | ±2.1 |
| | (8) | (8) | (8) | (8) | (8) | (8) | (8) |
| Acetyl | 14.5 | 15.8 | 14.7 | 15.6 | 14.1 | 15.2 | 14.9 |
| L-carnitine | ±0.9 | ±2.2 | ±1.5 | ±1.9 | ±2.0 | ±1.9 | ±1.6 |
| 100 mg/kg | (12) | (12) | (12) | (12) | (12) | (12) | (12) |

| | | | | | | | |
|---|---|---|---|---|---|---|---|
| Values are mean ± standard error. In brackets is reported the number of animals. | | | | | | | |

The acetyl L-carnitine, at a dose of 30 mg/kg, SC, had no analgesic effect on mice during the hot-plate test, both after acute administration (Table 3 (A)), and administration of 30 mg/kg BID SC for 7 days (Table 3 (B)).

**TABLE 3 (A)**

| SC acute treatment | | | | |
|---|---|---|---|---|
| Hot plate | | | | |
| Licking latency (sec) | | | | |
| | Before treatment | After treatment | | |
| | | 15 min | 30 min | 45 min |
| Physiological | 13.4±1.1 | 14.3±1.3 | 14.1±1.7 | 15.1±1.9 |
| solution | (10) | (10) | (10) | (10) |
| Acetyl L-carnitine | 14.2±0.9 | 15.8±1.8 | 16.5±2.0 | 15.0±2.1 |
| 30mg/kg | (15) | (15) | (15) | (15) |

| | | | | |
|---|---|---|---|---|
| Values are mean ± standard error. In brackets is reported the number of animals. | | | | |

**TABLE 3 (B)**

| 7-day sub-chronic treatment (SC) | | | | |
|---|---|---|---|---|
| Hot plate | | | | |
| Licking latency (sec) | | | | |
| | Before treatment | After treatment | | |
| | | 15 min | 30 min | 45 min |
| Physiological | 17.1±1.2 | 17.5±1.9 | 16.6±1.8 | 15.9±2.1 |
| solution | (10) | (10) | (10) | (10) |
| Acetyl L-carnitine | 15.8±1.3 | 16.3±2.1 | 17.3±1.9 | 15.2±1.5 |
| 30 mg/kg BID | (16) | (16) | (16) | (16) |

| | | | | |
|---|---|---|---|---|
| Values are mean ± standard error. In brackets is reported the number of animals. | | | | |

### EXAMPLE 2

The second experiment was the abdominal constriction test.

Male Swiss Webster mice weighing between 22 g and 30 g were used.

The abdominal constriction test was carried out according to Koster et al. in Fed. Proc. 18: 412, 1959.

According to this test, an IP injection of 0.6% acetic acid solution is to be made 5 minutes before starting the observation, which lasted 10 minutes.

In some experiments, the percentage of acetic acid was reduced by half (0.3%).

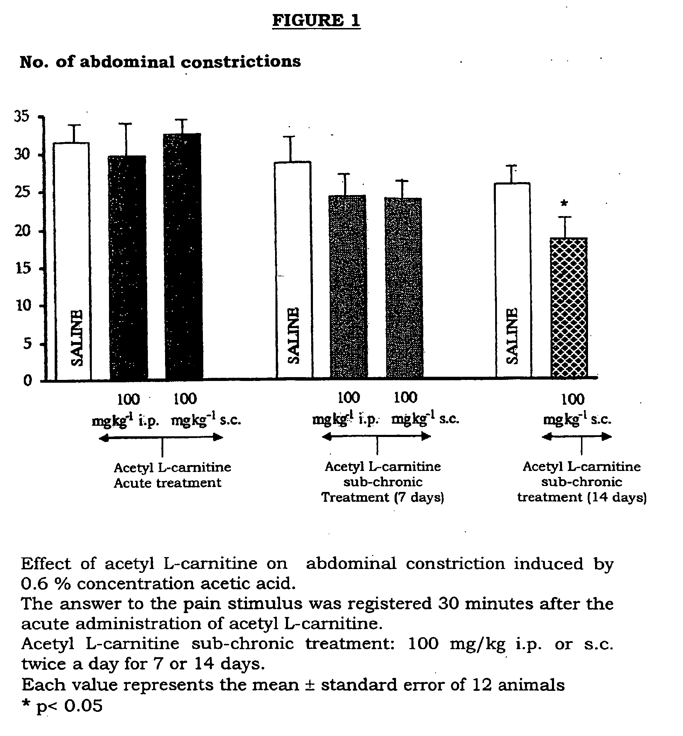

When the abdominal constriction test was carried out by using 0.6% acetic acid solution, it took 14 days of treatment before the acetyl L-carnitine exerted an antinociceptive effect (100 mg/kg BID, SC or IP), while both the acute administration (SC or IP), and the one repeated for 7 days, always at the same dose, had no effect at all (Figure 1).

Therefore, a 14-day administration was necessary to obtain analgesia in this particular test involving intense abdominal pain.

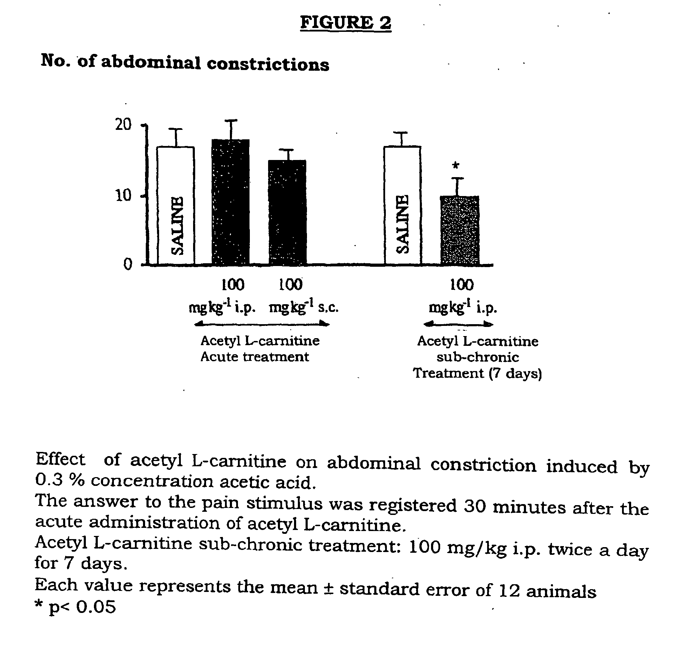

When the abdominal contractions were induced by 0.3% acetic acid, the statistic significance of the acetyl L-carnitine (100 mg/kg BID, IP or SC) was attained after a 7-day treatment (Figure 2).

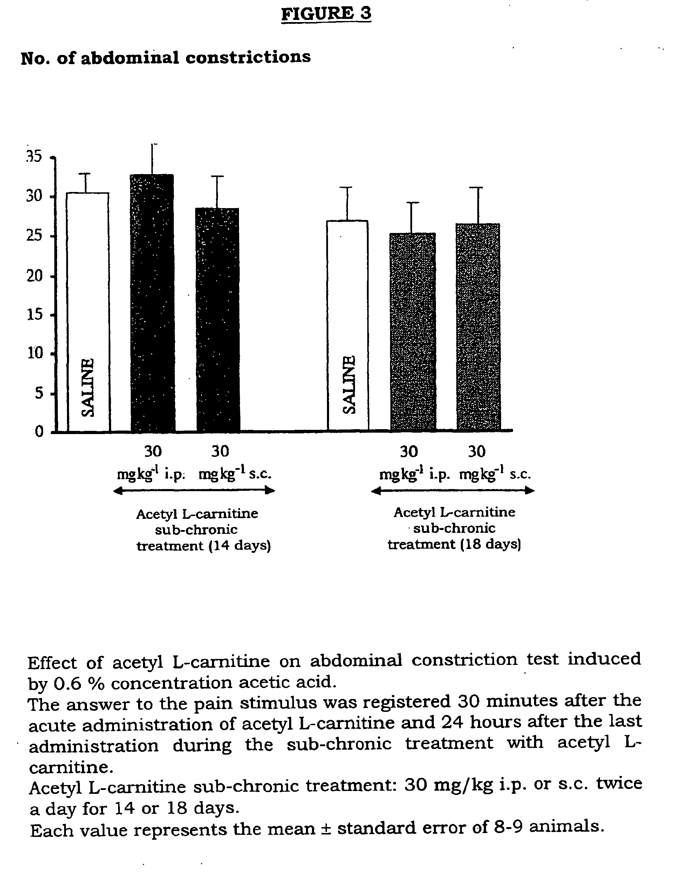

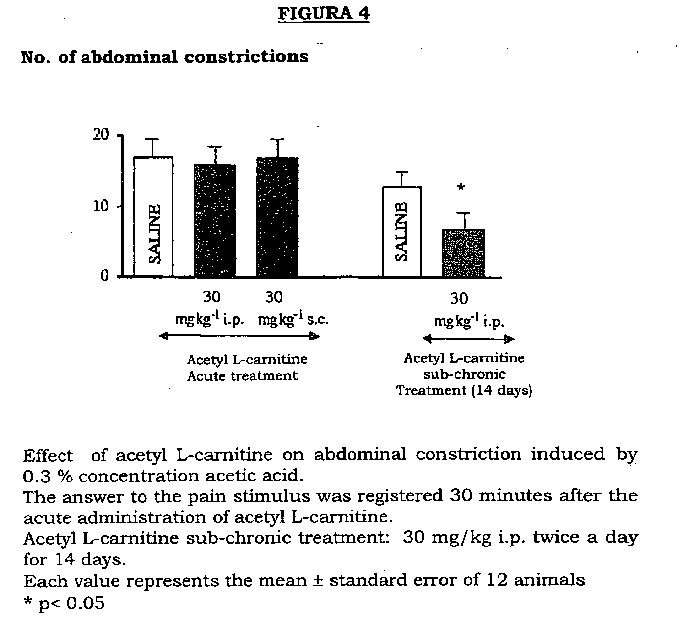

Similarly to what observed in the hot-plate test, the acetyl L-carnitine at a dose of 30 mg/kg IP (or SC) had no effect even after a 18-day treatment in presence of 0.6% acetic acid (Figure 3). Unlike what happened in the hot-plate test, the statistic significance was obtained after 14 days in presence of a small concentration of acid (Figure 4).

Both in the hot-plate test (Table 4 (A)), and in the abdominal constriction test (0.3 and 0.6%) (Table 4 (B) and (C), no increase in the pain threshold was observed in those mice receiving acetyl L-carnitine solution (3 mg/ml) dissolved in a bottle for 7 and 14 days:

**TABLE 4 (A)**

| 7 - 14 day sub-chronic treatment | | | |
|---|---|---|---|
| Hot plate | | | |
| Licking latency (sec) | | | |
| | Before treatment | After treatment | |
| | | 7 days | 14 days |
| Physiological | 14.4±1.0 | 16.6±1.9 | 17.2±2.2 |
| solution | (15) | (15) | (15) |
| Acetyl L-carnitine | 13.8±0.8 | 17.8±2.3 | 18.8±2.5 |
| 3 mg/ml in drinking water | (15) | (15) | (15) |

| | | | |
|---|---|---|---|
| Values are mean ± standard error. In brackets is reported the number of animals. | | | |

**TABLE 4 (B)**

| Number of constrictions (0.6% acetic acid) | | |
|---|---|---|
| Sub-chronic treatment | | |
| | 7 days | 14 days |
| Physiological | 33.1±3.6 | 25.8±4.8 |
| solution | (10) | (10) |
| Acetyl L-carnitine | 31.5±4.0 | 28.3±5.1 |
| 3 mg/ml in drinking water | (6) | (6) |

| | | |
|---|---|---|
| Values are mean ± standard error. In brackets is reported the number of animals. | | |

**TABLE 4 (C)**

| Number of constrictions (0.3% acetic acid) | | |
|---|---|---|
| Sub-chronic treatment | | |
| | 7 days | 14 days |
| Physiological solution | 14.5±2.6 | 12.7±3.4 |
| | (10) | (10) |
| Acetyl L-carnitine | 12.7±3.5 | 11.9±2.2 |
| 3 mg/ml in drinking water | (6) | (6) |

| | | |
|---|---|---|
| Values are mean ± standard error. In brackets is reported the number of animals. | | |

In this case, it must be considered that the quantity of solution drunk by the treated mice was roughly half of the normal quantity of water drunk by mice of the same weight.

### EXAMPLE 3

The analgesia induced by the acetyl L-carnitine (100 mg/kg BID, SC for 7 days) was determined in presence of some antagonists of some neurotransmission systems deeply involved in the regulation of pain threshold, and in particular the naloxone (opioid antagonist), the CGP-35348 (GABA_{B} antagonist) and the alphamethyl-p-tyrosine (inhibitor of the synthesis of cathecolamines).

These compounds were not capable to antagonize the analgesia induced by the acetyl L-carnitine (Table 5).

In agreement with the standard procedures known by the person skilled in the art, the doses used for any antagonist were the minimum required to prevent the analgesia induced by morphine (7 mg/kg SC) (1, 100 e 200 mg/kg IP), baclofen (4 mg/kg SC) and amphetamine (1 mg/kg SC) (non-reported data) respectively.

According to these experiments, it was possible to exclude that the opioid, GABAergic and catecholaminergic/serotoninergic systems were involved in the analgesia induced by the compound in question.

On the contrary the treatment with compounds having anticholinergic action, such as: atropine (non-selective muscarinic antagonist), S-(-)-ET-126 (0.1 µg/mouse ICV) (M₁ selective antagonist), pirenzepine (0.1 µg/mouse ICV) (M₁ selective antagonist) and hemicholinium-3 (HC-3) (1 µg/mouse ICV) (Depletor of acetylcholine by the block of the choline reuptake) have been found capable to antagonize the increase of the pain threshold induced by the acetyl L-carnitine (Table 5).

**TABLE 5**

| Hot plate | | | | | |
|---|---|---|---|---|---|
| Licking latency (sec) | | | | | |
| | | Before treatment | After treatment | After treatment | After treatment |
| Pre-Treat. | Treatment | | 15 min | 30 min | 45 min |
| Physiological solution 10 ml/kg | Physiological solution (PS) | 15.6±0.9 | 14.7±1.5 | 14.9±0.8 | 14.8±0.8 |
| Physiological solution 10 ml/kg | Acetyl L-carnitine | 21.8±1.1* | 22.6±1.3* | 22.5±1.3* | 22.9±1.6^ |
| Naloxone 1 mg/ kg | PS | 14.0±0.7 | 13.3±1.3 | 13.1±1.1 | 14.2±1.3 |
| Naloxone 1 mg/kg | Acetyl L-carnitine | 22.6±0.6* | 22.3±1.9* | 22.1±2.0* | 20.6±1.2^ |
| CGP 35348 100 mg/kg | PS | 14.4±0.8 | 12.4±1.0 | 12.6±1.3 | 12.2±1.6 |
| CGP 35348 100 mg/kg | Acetyl L-carnitine | 22.1±0.7^{*} | 21.1±1.2^ | 21.0±2.1^ | 20.3±1.9^ |
| Alpha-M-*p*-T 200 mg/kg | PS | 14.6±0.9 | 15.4±1.6 | 16.1±1.8 | 13.6±1.5 |
| Alpha-M-*p*-T 200 mg/kg | Acetyl L-carnitine | 22.0±1.3* | 22.5±2.0* | 21.5±1.7* | 21.6±1.5^ |
| Atropine 5 mg/kg | PS | 14.1±0.9 | 13.7±1.1 | 15.4±1.2 | 13.1±1.9 |
| Atropine 5 mg/ kg | Acetyl L-carnitine | 21.2±1.0^ | 11.4±1.7° | 12.6±1.9° | 12.8±1.3° |
| HC-3 1 µg ICV | PS | 14.1±0.9 | 13.7±1.1 | 15.4±1.2 | 13.1±1.9 |
| HC-3 1µg ICV | Acetyl L-carnitine | 22.0±1.1^ | 14.6±1.3° | 14.0±1.8° | 15.5±1.9° |
| Pirenzepine 0.1 µg ICV | PS | 13.6±0.8 | 13.9±1.5 | 14.5±1.8 | 15.5±1.5 |
| Pirenzepine 0.1 µg ICV | Acetyl L-carnitine | 21.6±1.3^ | 16.5±1.8° | 14.3±2.0° | 17.1±1.6° |
| S-(-)-ET-126 0.1 µg ICV | PS | 15.0±1.0 | 15.3±1.8 | 16.2±1.9 | 16.1±2.0 |
| S-(-)-ET-126 0.1 µg ICV | Acetyl L-carnitine | 22.9±1.3^ | 15.8±2.2° | 16.9±2.3° | 15.8±1.8° |

| | | | | | |
|---|---|---|---|---|---|
| Acetyl L-carnitine: 100 mg/kg BID, SC for 7 days. Values are mean ± standard error of at least 12 animals. ^ p<0.05; * p<0.01 compared with the group treated with PS. ° p<0.01 compared with the group treated with Acetyl L-carnitine. | | | | | |

The observation that the analgesia induced by the acetyl L-carnitine is mediated by the M₁ muscarinic receptor sub-type is confirmed by the application of the anti-sense strategy.

The administration of oligonucleotides against the gene coding for the M₁ (aODN-anti M₁) receptor at a dose of 3 nanomoles for each single intracerebroventricular injection (ICV) was found to be capable to prevent the production of the antinociception induced by the repeated administration of acetyl L-carnitine (100 mg/kg BID, SC for 7 days) (Table 6).

The idea of carrying out this series of experiments with aODN in the presence of acetyl L-carnitine comes from Ghelardini et al. (Br. J. Pharmacol., 128, 1633-1640, 2000), who demonstrated that both the physostigmine-induced analgesia, and the analgesia induced by direct cholinomimetics are completely prevented by a pre-treatment with aODN-anti-M₁ at a dose of 3 nanomoles/each single ICV injection.

In the same experimental conditions, the pre-treatment of the animals with the aODN (DOTAP) vehicle, as well as with the anti-sense degenerate oligonucleotide (dODN) (mixture of 3 x 10¹⁴ different oligonucleotides each, present in the site of action at a concentration lower than 10⁻¹⁸ M) was found to have no effect on the analgesia induced by acetyl L-carnitine (Table 6).

The data relating to the antagonism exerted by the hemicholinium-3, it is possible to deduce that the acetyl L-carnitine does not act as a direct muscarinic antagonist, but it enhances the functionality of the cholinergic system.

**TABLE 6**

| Hot plate | | | | | |
|---|---|---|---|---|---|
| Licking latency (sec) | | | | | |
| | | Before treatment | After treatment | After treatment | After treatment |
| Pre-treat. (ICV single injection) | Treatment | | 15 min | 30 min | 45 min |
| AODN 3 nmol | Physiological solution | 14.3±1.2 | 15.5±2.3 | 15.4±1.6 | 16.0±2.2 |
| AODN 3 nmol | Acetyl L-carnitine | 13.5±0.8* | 16.6±1.9* | 15.8±1.8* | 15.8±1.5* |
| dODN 3 nmol | Physiological solution | 13.3±1.4 | 12.9±2.4 | 14.1±2.5 | 15.3±2.1 |
| dODN 3 nmol | Acetyl L-carnitine | 23.6±1.1 | 22.6±2.3 | 21.9±1.5 | 22.3±1.7 |

| | | | | | |
|---|---|---|---|---|---|
| Acetyl L-carnitine: 100 mg/kg BID, SC, for 7 days. aODN was injected via ICV on days 1, 4 and 7; The nociceptive response has been recorded 24 hours after the last injection of Acetyl L-carnitine. Values are mean ± standard error of at least 10 animals. * p<0.01 compared with the group treated with dODN + Acetyl L-carnitine. | | | | | |

### EXAMPLE 4

The following tests have been carried out to determine the effects that acetyl L-carnitine may have on the nervous system: the rota-rod test, the hole board test and the sleep induction test.

Male Swiss Webster mice weighing between 22 g and 30 g were used.

The rota rod test was carried out by means of an apparatus consisting of a platform and a 3 cm-diameter rotating rod (16-20 r.p.m.) with a non-slipping surface.

The integrity of the animals' motor coordination was determined according to the capacity of the mice to keep their balance on the rotating rod.

At various intervals after the treatment, the parameter considered was the number of falls of the animal within a 30 second-observation (Vaught et a., Neuropharmacol., 24: 211, 1985).

In the hole board test, the rat's movements were recorded by two separate photocell systems, which recorded both the movements on the board (spontaneous motility) and how many times the mouse put its head into the holes on the surface (exploratory activity).

The mice were placed on the board one at a time and left free to explore both the surface and the holes for 5 minutes.

In the sleep induction test with pentobarbital, the duration of the loss of the writhing reflex was measured in the mice treated with pentobarbital (60 mg/kg IP).

Motor coordination of the mice treated with the acetyl L-carnitine (100 mg/kg BID, SC or IP for 7 and 14 days) was found not different from motor coordination of the control mice treated with physiological solution. The number of falls during the 30-second observation, in fact, decreased progressively as the sessions were repeated and this proves that the treated mice had learnt to keep their balance on the rotating rod:

**TABLE 7 (A)**

| 7-day sub-chronic treatment (IP) | | | | |
|---|---|---|---|---|
| **ROTA-ROD** | | | | |
| No. of falls in 30 seconds | | | | |
| | Before treatment | After treatment | | |
| | | 15 min | 30 min | 45 min |
| Physiological solution | 3.8±0.4 (5) | 2.0±0.2 (5) | 1.8±0.3 (5) | 0.8±0.2 (5) |
| Acetyl L-carnitine 100 mg/kg BID | 3.6±0.5 (5) | 2.2±0.4 (5) | 1.5±0.3 (5) | 1.1±0.3 (5) |

| | | | | |
|---|---|---|---|---|
| Values are mean ± standard error. In brackets is reported the number of animals. | | | | |

**TABLE 7 (B)**

| 14-day sub-chronic treatment (IP) | | | | |
|---|---|---|---|---|
| **ROTA-ROD** | | | | |
| No. of falls in 30 seconds | | | | |
| | Before treatment | After treatment | | |
| | | 15 min | 30 min | 45 min |
| Physiological solution | 4.2±0.4 (5) | 3.1±0.4 (5) | 2.2±0.2 (5) | 1.6±0.3 (5) |
| Acetyl L-carnitine 100 mg/kg BID | 3.9±0.5 (5) | 2.9±0.3 (5) | 1.7±0.4 (5) | 1.0±0.3 (5) |

| | | | | |
|---|---|---|---|---|
| Values are mean ± standard error. In brackets is reported the number of animals. | | | | |

The spontaneous motility and the exploratory activity of the treated mice, which were determined through the hole-board test, did not show any difference compared with the control group:

**TABLE 8**

| 14-day sub-chronic treatment (SC) | | |
|---|---|---|
| | **EXPLORATORY ACTIVITY** | **LOCOMOTOR ACTIVITY** |
| | No. of movements | No. of explorations |
| Physiological solution | 59.4±7.7 (7) | 21.3±5.6 (7) |
| Acetyl L-carnitine 100 mg/kg BID | 52.3±6.5 (10) | 24.0±5.1 (10) |

| | | |
|---|---|---|
| In brackets is reported the number of animals. | | |

Similarly to what observed in the two tests above, also in the case of the sleep induction test with pentobarbital (60 mg/kg IP) (administered 24 hours after the end of the treatments), the acetyl L-carnitine (100 mg/kg BID, SC for 7 days) neither affected the duration of sleep nor the induction times, unlike to what happened with the piracetam (30 mg/kg IP):

**TABLE 9**

| | **SLEEP** | |
|---|---|---|
| | Induction time (min) | Duration (min) |
| Physiological solution | 3.3±1.8 (16) | 24.2±6.0 (16) |
| Acetyl L-carnitine | 3.3±2.1(10) | 23.5±3.4(10) |
| Piracetam 30 mg/kg IP | 4.5±2.1(10) | 12.5±5.3* (10) |

| | | |
|---|---|---|
| Acetyl L-carnitine: 100 mg/kg BID, SC for 7 days. In brackets is reported the number of animals. * p<0.01 compared with the group treated with physiological solution. | | |

### EXAMPLE 5

The hyperalgesia induced by morphine withdrawal syndrome was assessed. Male Swiss Webster mice weighing between 22 g and 30 g together with male rats were used.

To induce dependence, the morphine was dissolved in the mice's bottle; the dose of morphine was increased every 48 hours.

The solution of morphine was sweetened with 5% sucrose to hide the bitter taste and was prepared according to the following schedule: 1^{st} and 2^{nd} day 0.1 mg/ml; 3^{rd} and 4^{th} day 0.2 mg/ml; 5^{th} and 6^{th} day 0.3 mg/ml; 7^{th} to 14^{th} day 0.4 mg/ml.

The 7-day treatment with acetyl L-carnitine started from day 7 of morphine treatment.

The morphine solution contained in the bottle was replaced with water on the morning of the 15^{th} day. Four hours after the solution had been replaced, the mice so treated showed during the hot plate test, a significant reduction in the pain threshold which reached its peak after 6 hours (11.8±0.8 seconds licking latency compared with 15.7±1.1 seconds recorded in the control group).

Motor coordination and the spontaneous activity assessed through the rota-rod test and the animex test respectively (Active 8 open field Activity System - Harvard Apparatus) did not appear to be different between the mice with morphine withdrawal syndrome and the mice treated with the sucrose solution used as a vehicle.

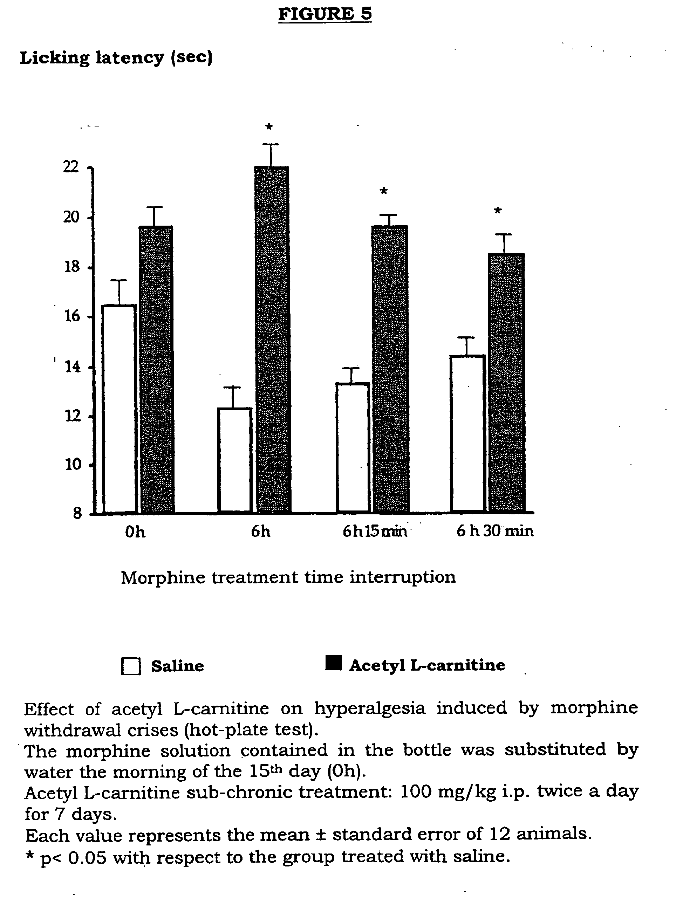

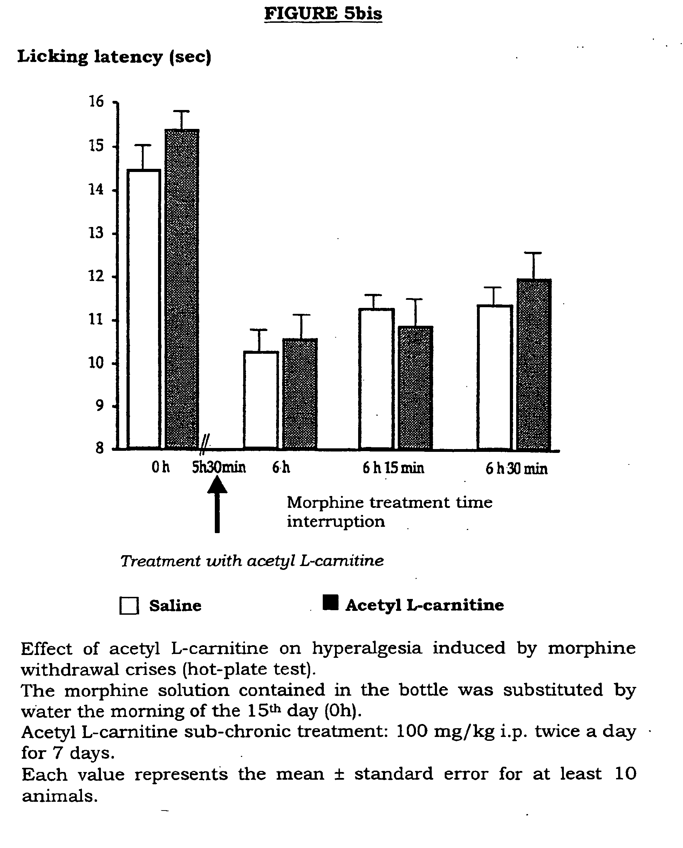

The acetyl L-carnitine was found capable of reverting the hyperalgesia induced by morphine withdrawal syndrome after the repeated treatment (100 mg/kg BID, IP or SC for 7 days) (figure 5), but not after one single administration (5 hours and 30 minutes from the replacement of the morphine with water) (figure 5bis).

### EXAMPLE 6

Male Swiss Webster mice weighing between 22 g and 30 g were used in the kainic acid-induced hyperalgesia and the test was carried out in compliance with the method described by Giovengo et al. in Pain, 83: 347-358, 1999.

According to this test, the hyperalgesia - determined in the mice through the hot plate test - is induced by a solution of kainic acid (20 mg/kg, IP).

The reduction in the pain threshold, which reached its peak 24 hours after the injection, persisted unchanged for a lot of days.

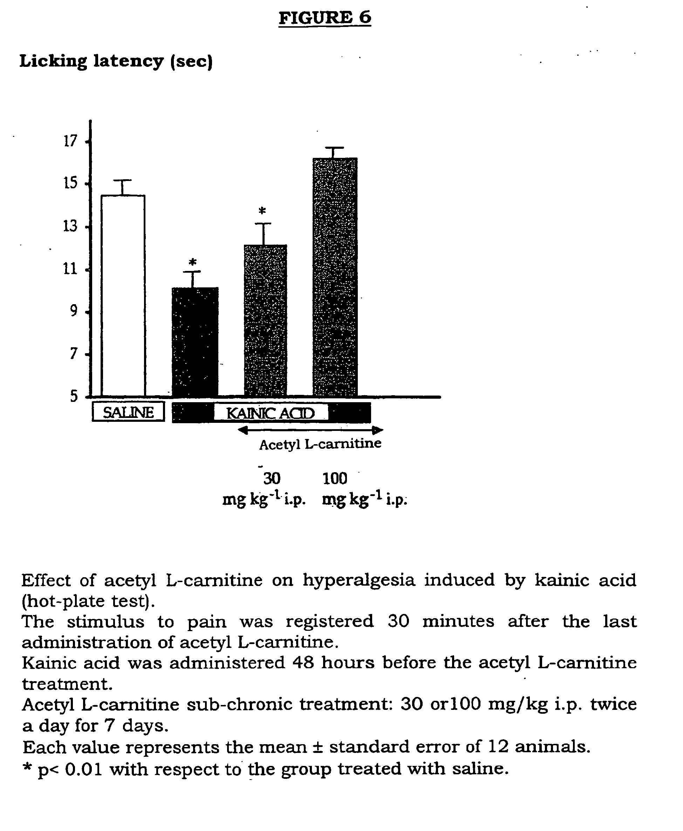

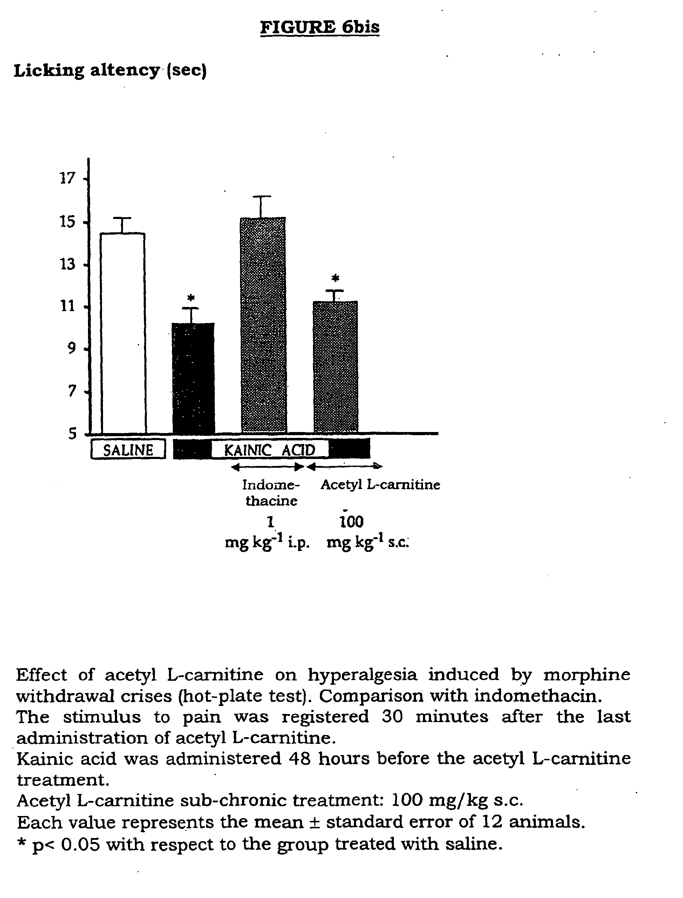

In this test, the 7-day repeated administration of acetyl L-carnitine (which started 48 hours after the administration of kainic acid) was found capable to revert the hyperalgesia (Figure 6), while the single administration was found to have no anti-hyperalgesic effect (Figure 6bis).

### EXAMPLE 7

In the test of hyperalgesia induced by NMDA (N-Metil-D-Aspartate), male Swiss Webster mice weighing between 22 g and 30 g were used.

This test was carried out in compliance with what described in the rats by Davies and Inturrisi in J. Pharmacol. Exp. Ther., 289: 1048-1053, 1999, but was applied to the mice by us.

According to this test, the hyperalgesia, assessed through the hot plate test, is to be induced by intrathecal administration of an NMDA solution (1.64 µg/mouse; administered around 15 minutes after the last administration of acetyl L-carnitine).

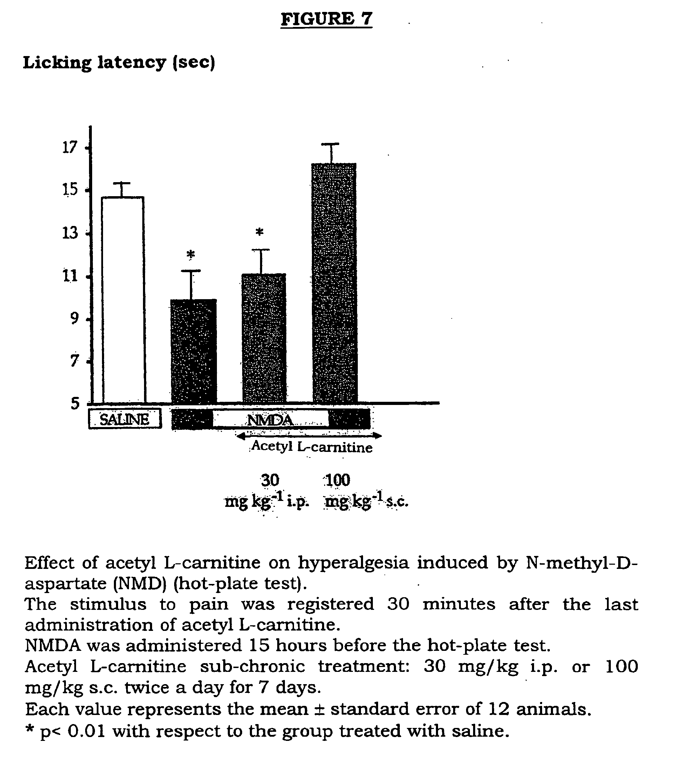

The reduction in the pain threshold, which reaches its peak 15 minutes after the injection of NMDA, disappears within 30 minutes from the administration. Figure 7 shows the anti-nociceptive effect exerted by the acetyl L-carnitine (100 mg/kg, BID, SC or IP for 7 days) in presence of the reduction in the pain threshold induced by NMDA.

In the same experimental conditions, the dose of 30 mg/kg BID, IP or SC for 7 days was not found to be active (figure 7) similarly to what can be observed in figure 6.

## Claims

1. Use of the acetyl L-carnitine, or a pharmaceutically acceptable salt thereof, for preparation of a medicament for the preventive therapy of post-surgical pain.

2. The use according to claim 1, wherein the acetyl L-carnitine salt is selected from the group consisting of: chloride, bromide, orotate, acid aspartate, acid citrate, citrate magnesium, acid phosphate, fumarate and acid fumarate, fumarate magnesium, lactate, maleate and acid maleate, mucate, acid oxalate, pamoate, acid pamoate, acid sulphate, phosphate glucose, tartrate, acid tartrate, tartrate magnesium, 2-amine ethansulphonate, magnesium 2-amine ethansulphonate, tartrate choline and trichloroacetate.

## Patentansprüche

1. Verwendung von Acetyl-L-carnitin oder einem pharmazeutisch akzeptablen Salz davon zur Herstellung eines Medikaments zur präventiven Therapie von postchirurgischen Schmerzen.

2. Verwendung nach Anspruch 1, worin das Acetyl-L-carnitinsalz ausgewählt ist aus der Gruppe, bestehend aus Chlorid, Bromid, Orotat, saurem Aspartat, saurem Citrat, Citratmagnesium, saurem Phosphat, Fumarat und saurem Fumarat, Fumaratmagnesium, Lactat, Maleat und saurem Maleat, Mucat, saurem Oxalat, Pamoat, saurem Pamoat, saurem Sulfat, Phosphatglucose, Tartrat, saurem Tartrat, Tartratmagnesium, 2-Aminethansulfonat, Magnesium-2-aminethansulfonat, Tartratcholin und Trichloracetat.

## Revendications

1. Utilisation de l'acétyl L-carnitine, ou de l'un de ses sels pharmaceutiquement acceptable, pour la préparation d'un médicament pour la thérapie préventive d'une douleur post-chirurgicale.

2. Utilisation selon la revendication 1, dans laquelle le sel d'acétyl L-carnitine est choisi dans l'ensemble consistant en : chlorure, bromure, orotate, aspartate acide, citrate acide, citrate de magnésium, phosphate acide, fumarate et fumarate acide, fumarate de magnésium, lactate, maléate et maléate acide, mucate, oxalate acide, pamoate, pamoate acide, sulfate acide, glucose phosphate, tartrate, tartrate acide, tartrate de magnésium, 2-amine éthanesulfonate, 2-amine éthanesulfonate de magnésium, tartrate de choline et trichloroacétate
